# EUROPEAN PATENT APPLICATION

(11) **EP 2 860 650 A1**
(43) Date of publication of application: **15.04.2015**
(21) Application number: 13187658.3
(22) Date of filing: 08.10.2013
(51) Int. Cl.: G06F 19/00

(54) **Computational approach for identifying a combination of two drugs**

(71) Applicant: Alacris Theranostics GmbH, 14195 Berlin (DE)
(72) Inventor: Kühn, Alexander, 10823 Berlin (DE); Lange, Bodo, 14169 Berlin (DE); Dreher, Felix, 10963 Berlin (DE); Peycheva, Svetlana, 10553 Berlin (DE); Lehrach, Hans, 14129 Berlin (DE); Wierling, Christoph, 14195 Berlin (DE)
(74) Representative: Fanelli Haag Kilger PLLC

(57) **Abstract**

The present invention relates to a method for identifying a therapeutic drug combination against a cancer, wherein the cancer comprises at least two alterations in at least two different, but crosstalking signaling pathways, the method comprising the steps of: a) providing a kinetic model of a biological network for said cancer comprising the at least two different, but crosstalking signaling pathways, wherein the kinetic model is generated by choosing a network topology, wherein the nodes of said topology represent biological entities selected from the group comprising genes, transcripts, peptides, proteins, protein modification states, small molecules, complexes, metabolites and modifications thereof, and the edges of said topology represent interactions between said entities, assigning kinetic laws and kinetic constants to the interactions and assigning concentrations to the biological entities, such that the kinetic model reflects the genome, epi-genome, proteome and/or transcriptome of said cancer, b)selecting test combinations from a plurality of known drugs, each test combination comprising at least two drugs, c) simulating the effect of each test combination on the biological network, thereby d) identifying from said test combinations a drug combination that acts against said cancer.

## Description

### FIELD OF THE INVENTION

The present invention is in the field of personalized medicine and systems biology, more in particular in the field of applying systems biology to the context of cancer therapy.

### BACKGROUND

Tumors are formed by rare, random changes in the genome or epigenome of somatic cells, which differ among every individual, allowing individual cells to escape the control mechanisms of the organism. Every tumor is therefore different, leading in consequence to response rates to the therapy as low as 25%. To complicate the situation further, tumors often are highly heterogeneous, either due to evolution of multiple parts of the tumor, and/or show cellular variations (e.g. tumor stem cells), potentially leading to a different response of individual cells in the same tumor to the therapy.

To be able to improve the prediction of the drug or therapy response of individual patients in view of this complexity, it is essential to make progress in two different directions: our capacity to carry out detailed molecular analyses of every tumor, as well as the development of techniques able to integrate large amount of molecular and other data. A major step in this direction has been the development of next generation sequencing (NGS) techniques, allowing large scale analysis of tumor/patient genomes, tumor epigenomes and tumor transcriptomes.

A wide range of different approaches have been taken to tackle the prediction of drug action based on genome data. One approach is the simple correlation of one or few biomarkers with published treatment outcome or the correlation with complex mutational or transcriptomic profiles. Other methods involve pattern matching or machine learning algorithms to find optimal drug treatment according to matching transcriptome or genome profiles.

This strategy does however have unavoidable limitations, since combinations of biomarkers are either highly correlated, and therefore only able to subdivide a patient population in two or few groups, or, if not, will define too many small groups, with most groups far too small to allow statistical analysis. More severely, it takes not into account complex data on the regulation and connectivity of cancer pathways.

The inventors hypothesize that the problem can realistically only be solved by computer models, able to build on the wealth of information generated by decades of cancer research, and the results of a detailed molecular analysis defining the exact form of the biological networks acting in the tumor and the other tissues of the patients. Hence, the application of systems biology approaches and predictive modeling enables to deal efficiently with the process of drug development and the personalized selection of drug treatment based on genomics/proteomics data.

To date, most studies on modeling the effects of cancer relevant drugs affecting the kinase signaling network are mostly confined to the modeling of single pathways. However, since tumors typically have thousands of somatic changes and are driven by many more mutations than previously thought, the inventors are convinced that single drug treatments are very often not sufficient to prevent tumor growth. Treatment strategies targeting at least two signaling pathways in parallel might provide an improved cancer treatment scheme, in particular due to suppression of arising resistance mechanisms in single agent treated cells.

Hence, there is a need to provide methods in order to identify such drug combinations. Further, there is a need for methods that can predict whether a particular drug combination efficiently acts against a specific cancer.

### BRIEF DESCRIPTION OF THE INVENTION

The present invention solves this problem by providing a method for identifying a therapeutic drug combination against a cancer, wherein the cancer comprises at least two alterations (e.g. mutations, overexpression, fusions, epigenetic changes and/or insertions) in at least two different, but crosstalking signaling pathways, the method comprising the steps of (a) providing a kinetic model of a biological network for said cancer comprising the at least two different, but crosstalking signaling pathways, wherein the kinetic model is generated by choosing a network topology, wherein the nodes of said topology represent biological entities selected from the group comprising genes, transcripts, nucleic acids (miRNA, ncRNA), peptides, proteins, protein modification states, small molecules, complexes, metabolites and modifications thereof, and the edges of said topology represent interactions between said entities, assigning kinetic laws and kinetic constants to the interactions and assigning concentrations to the biological entities, such that the kinetic model reflects the genome, epigenome, proteome and/or transcriptome of said cancer, (b) selecting test combinations from a plurality of known drugs, each test combination comprising at least two drugs, (c) simulating the effect of each test combination on the biological network, thereby (d) identifying from said test combinations a drug combination that acts against said cancer.

The problem is further solved by a method for predicting the response of a cancer to a therapeutic drug combination, wherein the cancer comprises at least two alterations (examples see above) in at least two different, but crosstalking signaling pathways, the method comprising the steps of (a) providing a kinetic model of a biological network for said cancer comprising the at least two different, but crosstalking signaling pathways, wherein the kinetic model is generated by choosing a network topology, wherein the nodes of said topology represent biological entities selected from the group comprising genes, transcripts, nucleic acids (miRNA, ncRNA), peptides, proteins, protein modification states, small molecules, complexes, metabolites and modifications thereof, and the edges of said topology represent interactions between said entities, assigning kinetic laws and kinetic constants to the interactions and assigning concentrations to the biological entities, such that the kinetic model reflects the genome, epigenome, proteome and/or transcriptome of said cancer, (b) providing a drug combination comprising at least two drugs, preferably one for each of the at least two different signaling pathways, (c) simulating the effect of the drug combination on the biological network, thereby (d) determining whether the cancer is responsive to the drug combination.

### DEFINITIONS

The term "therapeutic drug combination" herein means a composition comprising at least two therapeutic drugs that may optionally be provided along with further excipients. Each of the drugs is understood to be present in a therapeutically effective amount. The drug combination preferably consists of two therapeutic drugs. The drug combination may also comprise two, three, four, five, six, or even more therapeutic drugs.

The term "therapeutic drug" herein means a substance that is capable of acting against a cancer, which may mean that the drug inhibits the growth of the cancer and/or directly or indirectly leads to its death.

A signaling pathway describes cell changes that are induced by receptor activation. Different signaling pathways are thus governed by different receptors.

Crosstalk refers to instances in which one or more components of one signaling pathway affect another. This can be achieved through a number of ways with the most common form being crosstalk between proteins of signaling pathways. In these signaling pathways, there are often shared components that can interact with either pathway. For example, crosstalk between proteins can be seen between cyclic adenosine monophosphate (cAMP) and mitogen-activated protein (MAP) kinase pathway in the regulation of cell proliferation.

Crosstalk can also be observed across membranes. Membrane interactions with the extracellular matrix (ECM) and with neighboring cells can trigger a variety of responses within the cell. For example, binding of the α5β1 integrin to its ligand (fibronectin) activates the formation of fibrillar adhesions and actin filaments. Yet, if the ECM is immobilized, matrix reorganization of this kind and formation of fibrillar adhesions is inhibited. In turn, binding of the same integrin (α5β1) to an immobilized fibronectin ligand is seen to form highly phosphorylated focal contacts/focal adhesion (cells involved in matrix adhesion) within the membrane and reduces cell migration rates.

Another example of crosstalk between two signaling pathways can be observed with the interaction of the cAMP and MAPK signaling pathways in the activation of lymphocytes. In this case, components of the cAMP pathway directly and indirectly affect MAPK signaling pathway meant to activate genes involving immunity and lymphocytes.

Crosstalk may further occur on a transcriptional level. For example, EGFR signaling silences proteins acting as negative regulators of Hedgehog (HH) signaling, as AKT- and ERK-signaling independent process. EGFR/HH signaling maintains high GLI1 protein levels which contrasted the GLI1 downregulation on the transcript level.

The herein disclosed method enables drug identification against a cancer. The cancer in the context of the application may be selected from the group consisting of carcinoma, sarcoma, lymphoma, leukemia, germ cell tumor and blastoma.

The cancer in the context of the present application is **characterized in that** it comprises at least two alterations. It is preferred that the alterations are in functional correlation with the cancer, e.g. determine cell control and/or growth. Preferably, the mutations occur in tumour-suppressor genes and/or oncogenes. The mutations may also embrace epimutations, i.e. epigenetic alterations, such as changes in DNA methylation and histone modification.

Preferably one of said at least two mutations occurs in a first signaling pathway, another mutation is present in a second signaling pathway. More preferably, the first and second signaling pathways are in crosstalk with each other. The at least two signaling pathways may be present in a single cell, or they may be present in different cells provided that crosstalk occurs across said cells.

### DETAILED DESCRIPTION OF THE INVENTION

The present invention relates to a method for identifying a therapeutic drug combination against a cancer, wherein the cancer comprises at least two alterations (e.g. mutations, overexpression, fusions, epigenetic changes and/or insertions) in at least two different, but crosstalking signaling pathways, the method comprising the steps of (a) providing a kinetic model of a biological network for said cancer comprising the at least two different, but crosstalking signaling pathways, wherein the kinetic model is generated by choosing a network topology, wherein the nodes of said topology represent biological entities selected from the group comprising genes, transcripts, nucleic acids (miRNA, ncRNA), peptides, proteins, protein modification states, small molecules, complexes, metabolites and modifications thereof, and the edges of said topology represent interactions between said entities, assigning kinetic laws and kinetic constants to the interactions and assigning concentrations to the biological entities, such that the kinetic model reflects the genome, epigenome, proteome and/or transcriptome of said cancer, (b) selecting test combinations from a plurality of known drugs, each test combination comprising at least two drugs, (c) simulating the effect of each test combination on the biological network, thereby (d) identifying from said at least two test combinations a drug combination that acts against said cancer.

The invention further relates to a method for predicting the response of a cancer to a therapeutic drug combination, wherein the cancer comprises at least two alterations (examples see above) in at least two different, but crosstalking signaling pathways, the method comprising the steps of (a) providing a kinetic model of a biological network for said cancer comprising the at least two different, but crosstalking signaling pathways, wherein the kinetic model is generated by choosing a network topology, wherein the nodes of said topology represent biological entities selected from the group comprising genes, transcripts, nucleic acids (miRNA, ncRNA), peptides, proteins, protein modification states, small molecules, complexes, metabolites and modifications thereof, and the edges of said topology represent interactions between said entities, assigning kinetic laws and kinetic constants to the interactions and assigning concentrations to the biological entities, such that the kinetic model reflects the genome, epigenome, proteome and/or transcriptome of said cancer, (b) providing a drug combination comprising at least two drugs, preferably one for each of the at least two different signaling pathways, (c) simulating the effect of the drug combination on the biological network, thereby (d) determining whether the cancer is responsive to the drug combination.

To integrate complex network information for the prediction of drug response, the inventors have developed a computer-implemented model reflecting a 'virtual patient' consisting of individual (appropriately compartmentalized) models of every relevant cell type (one or more tumor cell type, possibly tumor stem cell, liver cells, normal patient cell types to be able to predict specific types of drug side effects etc.), exchanging appropriate signals.

In the context of the present invention the biological network represents one or more cells, a tissue or a cell line-. In a preferred embodiment the biological network represents a human or a part thereof.

To provide data for said complex network it is necessary to analyze the at least parts of the genome, epigenome, transcriptome and/or proteome of said cancer. Preferably this analysis is able to determine at least two alterations.

The analysis of the genome, epigenome, transcriptopme and/or proteome may be performed with any suitable method, non-limiting examples are sequencing or mass spectrometric analysis. In a preferred embodiment the genome, epigenome and/or transcriptome are analysed by sequencing, preferably by next-generation sequencing.

### Kinetic model

The general design of the kinetic model is outlined below. For further details on the model, the reader is referred to WO2010/025961A2 and the further publications referenced in the following.

The term "model" as used herein refers to an in silico representation of a biological system. A "kinetic model" is a model capable of describing the time-dependent behavior of a biological system. Necessary ingredients for predicting the time-dependent behavior include kinetic laws and associated kinetic constants governing the interactions between constituents of the biological system including the conversion of constituents of the biological system. These constituents are herein also referred to as "biological entities".

The term "biological entity" comprises any molecule which may occur in a biological system. Preferred biological entities are biomolecules which are further detailed below. The biological entities render the model an in silico representation of a biological system, in the present case a "virtual patient". The model according to the invention furthermore comprises starting concentrations of the biological entities. Kinetic laws, kinetic constants and starting concentrations together permit the prediction of the time dependent behavior of said biological network. The term "assigning" refers to fixing or setting certain properties or numeric values at the beginning of the simulation. While kinetic laws and kinetic constants preferably do not change during the simulation, it is self-evident that concentrations of the biological entities as assumed during the simulation may differ from the respective starting concentrations.

The biological systems this invention pertains to are biological networks comprising signaling pathways.

Networks may be referred to and represented as "graphs". More specifically and as well known in the art, a network or graph comprises nodes and edges. Nodes and edges together form the topology of the network. The nodes of said network are the in silico counterparts of the above mentioned biological entities and the edges of said network are the in silico counterparts of interactions between the above mentioned entities. The term "interactions" as used herein refers to any kind of interactions, in particular to those interactions which may affect the concentrations of the biological entities involved in said interaction. More specifically, the term "interaction" includes conversion of one or more given biological entities into one or more different biological entities, possibly under the influence of one or more further biological entities. For example, active Ras in the MAPK pathway is generated from inactive Ras by binding to Guanosine-5'-triphosphate (GTP). Other preferred interactions include decrease or increase of the amount or concentration of one or more biological entities, for example as a consequence of the action, presence or absence of one or more other biological entities. For example, mitogen-activated protein kinase (MAPK) interacts with C-myc in the MAPK pathway by adding a phosphate group, thereby increasing the concentration of phosphorylated C-myc. Yet another preferred interaction is the formation of a complex from two or more biological entities.

In other words, the interactions according to the invention involve or entail reactions. Reactions according to the invention may be modeled using mass action kinetics but can, in general, follow any other suitable kinetic law. As is well-known in the art, mass action kinetics depends on the concentrations of the biological entities involved in a given reaction and the kinetic constants.

Modeling the kinetics of a biological system requires knowledge of all kinetic laws, kinetic constants and (starting) concentrations of all involved biological entities or reactants. However, the exact values of specific parameters (kinetic constants, starting concentrations of components) can often not be directly measured. This problem can be overcome by a Monte Carlo-based approach, in which such unknown parameters are drawn from probability distributions, reflecting our knowledge (or lack of knowledge), generating random parameter vectors, each of which is then used to model all the different states we want to compare (e.g. tumor or patient without treatment, with all possible treatments or treatment combinations etc.).

Further, experimental data for some starting concentrations may be obtained by performing measurements in the naturally occurring counterpart of the biological network to be simulated, i.e. for example in cells or cell lines.

The mathematical concepts and the methodology underlying the present invention are also described in detail in WO2010/025961A2 as well as the publication Kühn et al. (2009). WO2010/025961A2 and the publication Kühn et al. (2009), and in the present context in particular the section entitled "Methods" of Kühn et al. (2009), is fully incorporated by reference.

### Cancerous network

The kinetic model used in the context of the invention reflects a diseased network, meaning that the alterations (e.g. chromosomal, genetic, epigenetic and/or transcriptional) of said cancer as compared to a normal network are considered. Such alterations may result from mutations, under- or overexpressions, fusions, epigenetic changes and/or insertions of a biological entity. Depending on the type of alteration, an interaction, kinetic law, kinetic constant and/or concentration is changed in the model. In preferred embodiments of the invention the alternations are gain of function, loss of function or gene-overexpression like.

In preferred embodiment, the effect of the mutation on the biological entity is modeled as known from literature or using inferences from bioinformatics technologies. For instance a silent mutation or a missense muation with no functional consequences are effectively modeled by the wild type biological entity, a missense mutation leading to a truncated form of the biological entity can be often modeled by the complete knock down (0%) of the biological entity, missense mutations that damage known functional domains can be modeled by removing the appropriate edge between the modeled biological entity and the biological entity the damaged domain was meant to interact with, constitutively activating mutations can be modeled by adding an artificial non-reversible reaction (edge) that converts the inactive form of the biological entity into the active form, and finally mutations which are known to change the enzymatic efficiency of an enzymatic biological entity are modeled by multiplying the kinetic constant by the known factor of change of efficiency; in all these cases the kinetic constants are either experimentally determined or are selected from a lognormal distribution.

In addition, it is preferred that active disease state data as embodied in gene expression, protein and phosphoprotein concentration, metabolite and micro-RNA levels are directly applied to the model by setting the initial concentrations of the appropriate biological entities to the levels described empirically.

### Crosstalking signaling pathways

Tumors often escape a monotherapy due to additional mutations in another pathway which may redirect the signaling cascade, thereby rendering the effect of the single drug almost ineffective. A combination therapy targeting both pathways could in this case desirable.

Hence, the inventors included into the kinetic model a so-called crosstalk between different signaling pathways. A crosstalk between the signaling pathways may occur via a protein shared by the signaling pathways, transmembrane crosstalk or crosstalk in transcriptional activation. The kinetic model may reflect said crosstalk by biological entities shared by the signaling pathways.

### Effects of drugs on cancerous network

To simulate the effect of a drug or drug combination on the biological network, the model must consider the interaction of said drug(s) on the network. It is therefore preferred that the method simulates the effect of a single drug of the selected drugs and/or determines the effectiveness of the drug combinations and compares the effect of the effect of the combination the the sum of the effectiveness of the single drugs corresponding to said combination. It is therefore preferable if the selected drugs have a known pharmacologic profile and/or preferably have a known IC50 value.

The described invention is suitable for mechanistical drugs. In a preferred embodiment of the invention the at least two drugs are targeted mechanistic drugs, in a more preferred embodiment these are selected from the group comprising tyrosinase kinase inhibitors and monoclonal antibodies.

For example, if the drug acts by inhibiting the activity of one or more biological entities, the drug action is modeled by a complex formation reaction of the drug and its target. The binding affinity of this binding reaction is set according to the experimentally defined K_{d} value of the drug-target interaction. Resulting complex lacks the biological activity of the unbound biological entitiy.

The modeled cellular concentration is generally considered to be the concentration of application. For instance, to model 500 nM of drug application, the cellular concentration is generally assumed to be 500 nM. However, if factors are known about the modeled drug e.g. permeability or solubility or about the modeled cell e.g. upregulated PGP or MDR-1 that would affect drug pharmacology, the modeled cellular concentration can be set to a fraction of the applied concentration; if this is done, it is preferably based on empirical data.

As the results of said invention are mostly theoretical it would be necessary to test the identified drug combination in a cancer specific cell line, a xenograft model and/or clinical trials.

### Initial conditions

In one embodiment, initial conditions comprise (a) experimentally determined concentrations of biological entities; and/or (b) experimentally determined mutation data.

In a further preferred embodiment of the methods according to the invention, said entities are biomolecules, preferably selected from nucleic acids including genes; (poly)peptides including proteins; small molecules; and complexes and metabolites of biomolecules. Small molecules include saccharides, amino acids, lipids, nucleotides, nucleosides as well as metabolites and derivatives thereof.

The biological entities may be genes, transcripts, peptides, proteins, protein modification states, small molecules (e.g. hormones, second messenger compounds), complexes, metabolites or modifications thereof.

Due to the complexity of cancer even within same types of tumors (i.e. heterogeneity) it has been challenging to gain insight into the real functional consequence of complicated mutational profiles. Ideally, meaningful information for defining personalized/individualized drug treatment also takes into account the expression profile (or complementary omics and proteomics data) to allow for prediction of the actual consequence of the genetic changes in the cancer cell that are likely to be reflected in the transcriptome. The kinetic model shall thus reflect the genome, epigenome, proteome and/or transcriptome of the cancerous network modeled.

The present invention furthermore provides a computer program adapted to perform the method of any one of the preceding claims.

Furthermore provided is a computer-readable data carrier, comprising the program according to the invention. Also provided is a data processing apparatus comprising means for performing the methods according to the invention or having a program according to the invention installed thereon.

### EXAMPLES

### Material and Methods

**Selection of cell lines.** We selected 5 major prevalent tumor types for computational modeling: intestine, liver, lung, prostate, and skin. For each of these tumor types we selected 2 different cell lines; in the case of skin cancer, we selected 10 melanoma cell lines. Therefore, in total we processed 18 cancer cell lines (intestine: COLO201, COLO205; liver: HEPG2, JHH4; lung: HCC78, NCIH647; prostate: 22RV, PC3; skin: A2058, A375, C32, CHL1, HS695T, HS936T, HT144, K029AX, SKMEL30, WM983B). The selection of specific cell lines out of all CCLE cell lines was conducted according to the overlap to our model, i.e. we chose those cell lines that had the highest overlap between its mutated genes and the model genes.

**Statistical analyses.** Affymetrix Human Genome U133 Plus 2.0 GeneChip arrays provided by CCLE were processed using R version 2.15.1. The complete set of arrays was normalized together using GCRMA and the mapping of probes to genes is based on ENSEMBL 65 using custom CDF version 15. For statistical analyses and results visualization R 2.15.1 were used.

**Drug inhibition curves and prediction accuracy.** Relative drug inhibition was calculated based on c-Myc-steady-state values as follows: first, we calculated the geometric mean and the standard deviation of the 10 Monte Carlo-based repeated measurements. Second, we calculated geometric mean ratios with the control sample as denominator. Third, the tumor vs. control ratio was defined as reference, and for the drug treatment samples, the relative reduction in comparison to this reference was calculated and visualized as 8 point drug inhibition curves.

In order to assess the results of the modeling, we defined 4 prediction accuracy categories: 1 = very good accuracy; 2,3 = good accuracy; 4 = poor accuracy. The categories were defined as follows:
1: Both the slopes and the measurements matched (a combination of categories 2 and 3)
2: at least 5 out of 8 data points (drug concentration measurements) matched. A match was defined as either the two points differed by less than 10% drug inhibition or the two points were located within the range of their error bars.
3: The absolute difference of the slopes of the regression lines differed by less than 10.
4: Neither the individual data points nor slopes did match.

In addition, we calculated two other parameters of the drug inhibition curves: the maximum inhibition value and the IC50 value. The maximum inhibition is defined as the lowest relative inhibition value out of the 8 drug dosages. The IC50 value is defined as the drug concentration where the inhibition reaches 50% (i.e. where the c-Myc ratio is half of the value in comparison to the untreated tumor). This was calculated as follows: if 50% inhibition was not reached in any of the 8 drug dosages, the IC50 value was set to the maximum concentration (8 µM). Otherwise, the IC50 value was calculated by linear regression through the two dosage points adjacent to the 50% inhibition.

**Model description.** The mathematical model used for drug sensitivity predictions covers major cancer-related signal transduction pathways and known transcriptional targets and is based on the model previously described (Rohr et al. PLoS One 8, e67461 (2013)). By rigorous literature screening using different resources, like KEGG, Reactome and ConsensusPathDB several pathways were expanded to include additional activating ligands, such as AREG, EREG, IGF2, IL13 and IL14. Additional signaling pathways, such as mTOR, protein kinase A (PKA), protein kinase C (PKC), melanocortin receptor 1 (MC1R) and macrophage stimulating protein 1 (MST1), were integrated using PyBioS, a web-based software for the modeling and simulation of cellular reaction systems. Moreover, mutated forms of about 60 oncogenes and tumor suppressor genes were implemented. Based on information from primary literature and several databases, like COSMIC a mutation database was set up which links these mutated forms to more than 400 different mutations (loss of function, gain of function and fusion) allowing to simulate their molecular effects. In addition, over 70 inhibitors and their corresponding target binding reactions were integrated into the model. Based on knowledge from textbooks, primary literature and several databases we generated a drug database that contains the main targets of every drug as well as the binding affinities of a drug to its targets was established. It is linked to the large cancer model and allows the simulation and prediction of drug effects on given cell lines.

Table 1 gives an overview on the effects of more than 80 different drugs (anti-cancer as well as non-anti-cancer drugs), which can be simulated taking more than 95 different drug targets into account.

In total, the expanded model covers 609 human genes corresponding to 3397 components connected by 5456 reactions. The respective ordinary differential equation (ODE) model has 5968 kinetic parameters, 2489 variables and 908 components that are treated as fixed.

**Monte Carlo modeling approach**. The reactions involved in the model consist of a small number of different reaction types such as synthesis reactions, product formation and degradation reactions that are described by irreversible mass action kinetics. Reversible reactions, as for example complex formation reactions are described by reversible mass action kinetics. Synthesis and decay reactions have been introduced where appropriate. The Monte Carlo modeling approach focuses on predicting changes in the concentrations of the model components given certain mutation patterns and expression values of the individual cell lines. Therefore, a cell line state (stimulation with growth factors, mutations, but no drugs) or a treated cell line state (stimulation with growth factors, mutations and different drugs or drug combinations and different concentrations) respectively, is compared with a control state (no growth factors, no mutations, no drugs). To compensate for the uncertainty in many of the parameters, the components of the parameter vector are chosen from appropriate probability distributions, reflecting available knowledge and each parameter vector is used to model all the individual cell lines. This approach was repeated 10 times for the control and cell line state, respectively. Subsequently, for the simulation results of each individual sampled kinetic parameter set, ratios were computed for all the components of the model and, finally, geometric mean values of the ratios were computed for each component over all Monte Carlo simulation runs of a given sample set (cell line state vs. control state or treated cell line state vs. untreated cell line state).

**Cell lines and pharmacological characterization**. Cell line HS695T (CLS) was cultured in DMEM (4mM L-glutamine, 4.5g/l glucose, Invitrogen) with 10% fetal bovine serum (Biochrom) and maintained at 37°C under 5% CO₂ atmosphere. The cells were dispensed as triplicates into a 6-well-plate-formate with a concentration of 5x10⁵ per well 24 hours prior to drug treatment. Afterwards medium was replaced with a final drug concentration range of 2.5nM to 8µM by 3.16-fold dilutions (eight-dose response) of Sunitinib and PI103 as single treatment HS695T for 72 and 96 hours. Rapamycin/Sirolimus and U0126 were combined in a double treatment for cell line HS695T with the lowest concentration of one compound supplemented with increasing concentration of the other and increasing concentration of both compounds in one treatment for 72 hours. Compounds (Selleckchem) were diluted in DMSO. The final DMSO concentration for the experiments was under 0.4%. Cell viability was determined via Trypan blue staining in duplicates for each well. 100µl of cells were incubated with an equal volume of 0.5% Trypan blue for 2-5 minutes (in 0.9% sodium chloride) and counted using a Neubauer haemocytometer chamber and a light microscope. The means of independent cell counts were taken for analysis.

### Results

### Example 1

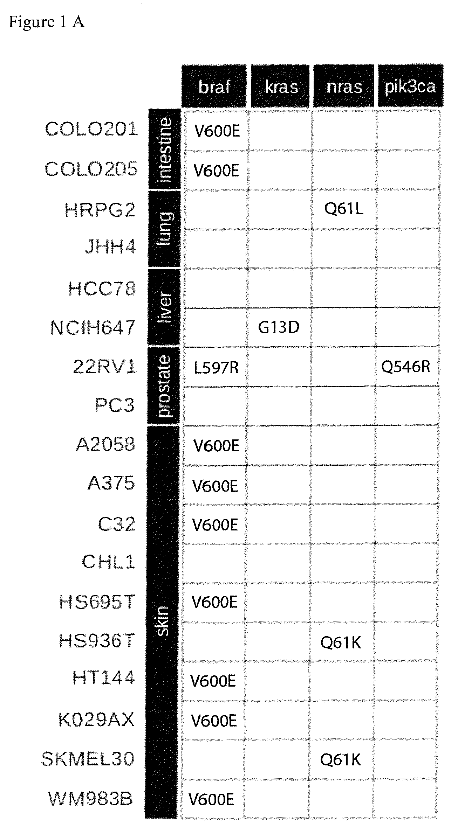

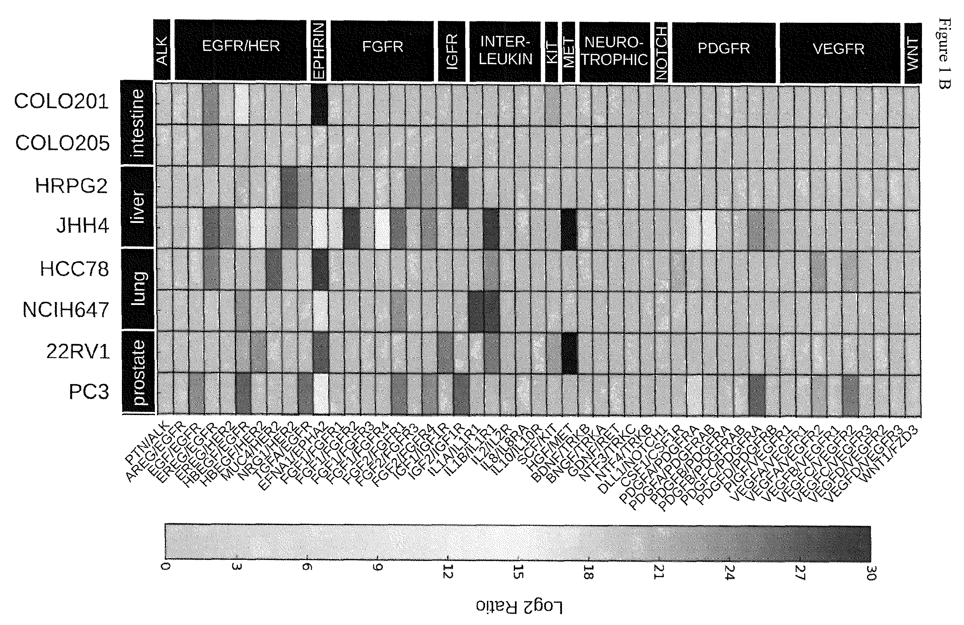

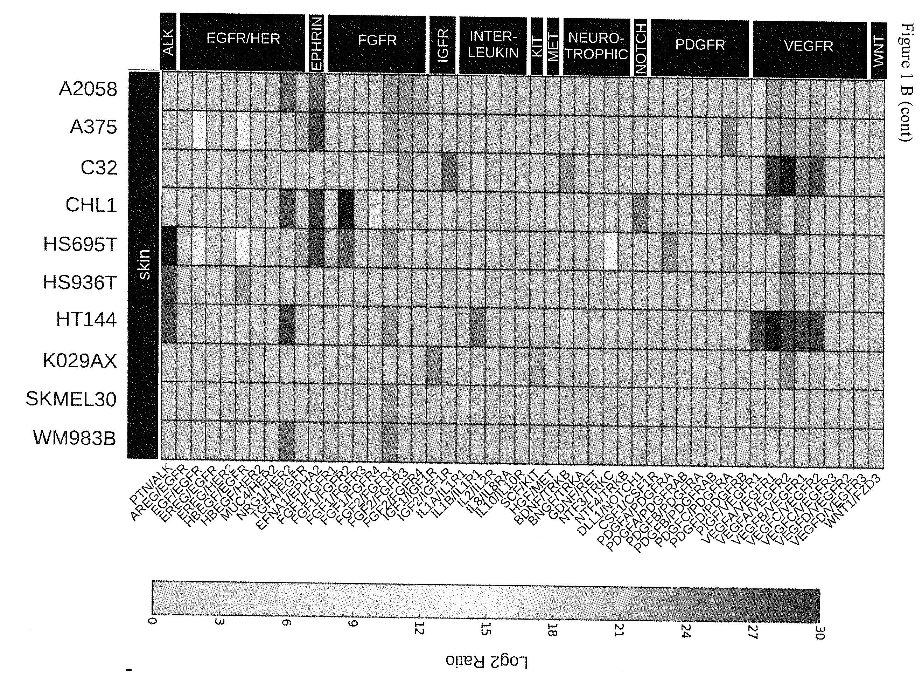

Individual in silico models of 18 selected cancer cell lines were generated. The cell lines were selected to include a range of different tissue type origin (skin, lung, prostate, liver, intestine), different pathway activity states and spectrum of mutations covered currently by the model (Fig. 1). A generic mathematical cancer model was generated for each cell line with cell line specific data on gene expression and somatic mutations for each cell line. Mutations were introduced into the model by gain-of-function effects for oncogenes according to information in databases. In total, within the selected cancer cell lines we identified 6 different mutations in 4 different oncogenes that were covered by our generic cancer model and consequently have been taken into account as activating mutations (Fig. 1A). Normalized relative gene expression values were used for the initialization of the synthesis rates of the corresponding proteins within the model reflecting the differences in expression for the cell lines. Subsequently, the Monte Carlo-based sampling approach was applied to analyze the individual cancer cell line characteristics by comparing a cell line state with a corresponding control state.

The results show predictions of changes in selected ligand-receptor-complexes of the model indicative for active pathways (e.g. EGF:Phospho-EGFR for active EGF signaling, DDL1:Notch1 for active Notch signaling etc.) for the 18 selected cancer cell lines modeled according to their expression profiles and mutation patterns (Fig. 1B). Although selected cancer lines show very similar patterns of implemented mutations, simulation shows a very heterogeneous pattern of active pathways within the cell lines. Some of the pathways seem to be mainly active in certain types of tumors: Signaling by VEGFs and neurotrophic factors for example seems to be mainly upregulated for melanoma cancer cell lines (except cell lines SMEL30 and WM983B), EGFR signaling in contrast is frequently activated in intestine, lung and liver cell lines. However, many signaling pathways, such as Ephrin, EGFR, FGFR and PDGFR, seem to be active in essentially all tissue types, but not in all cell lines of a tissue type. Some pathways, such as ALK, KIT and MET signaling, are only active in specific single cell lines. For example, the cell line CHL1 is the only melanoma cell line where the Delta/Notch pathway was found activated. This analysis shows that the cancer cell lines subjected to our analysis show diverse patterns of pathway activation even when they were selected from the same histopathological classification (Fig. 1B).

### Example 2

The individual cancer cell line models were employed to model the drug action of 12 molecular targeted drugs for which pharmacological profiles were available from CCLE (Barretina et al. Nature 483, 603-607 (2012)). To generate predicted growth inhibition curves, we simulated a concentration range of 8 µM to 2.5 nM (8 point dose response) by 3.16-fold dilutions for every compound. Inhibitor components in the model as well as k_{D} values of corresponding inhibition reactions were initialized according to desired concentration and to information in drug databases that contains the main targets (and if available the off-targets) of every drug as well as the binding affinities (k_{D} values) of a drug to its targets.

After simulation for each parameter setting, the final steady state concentration ratio (cell line state vs. control state and treated cell line state vs. control state, respectively; c-Myc was computed as a surrogate marker for cell proliferation. This yielded a series of concentration ratios for the cell line state and for every drug treatment across the cell line panel. Finally, concentration ratios of c-Myc for each drug treatment were normalized to give a c-Myc ratio for cell line state. For each compound across the panel of cell lines growth inhibition curves were generated by plotting normalized c-Myc ratios against drug concentration. Predicted growth inhibition curves were compared to growth inhibition curves determined by CCLE and prediction accuracy was calculated as described in Material & Methods. Essentially 4 different categories of prediction accuracy were defined to assess the results of the modeling based on the slope of the 8 point response curve and the proximity of measured and predicted data points.

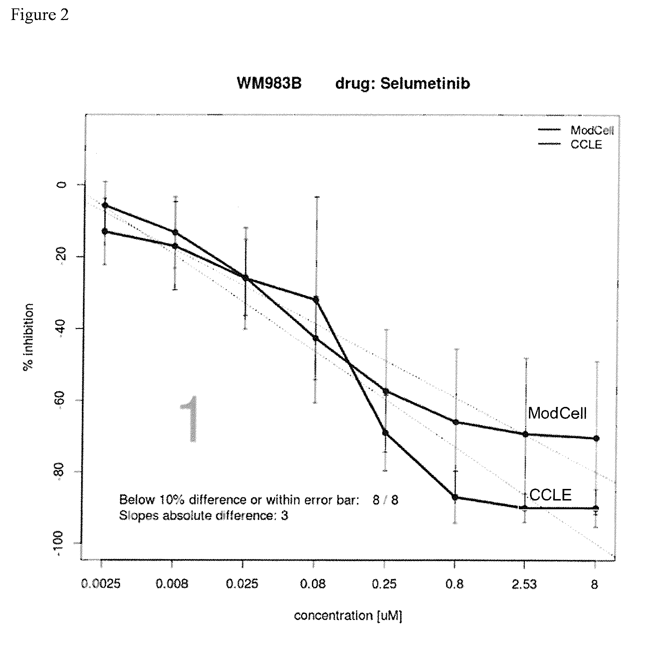

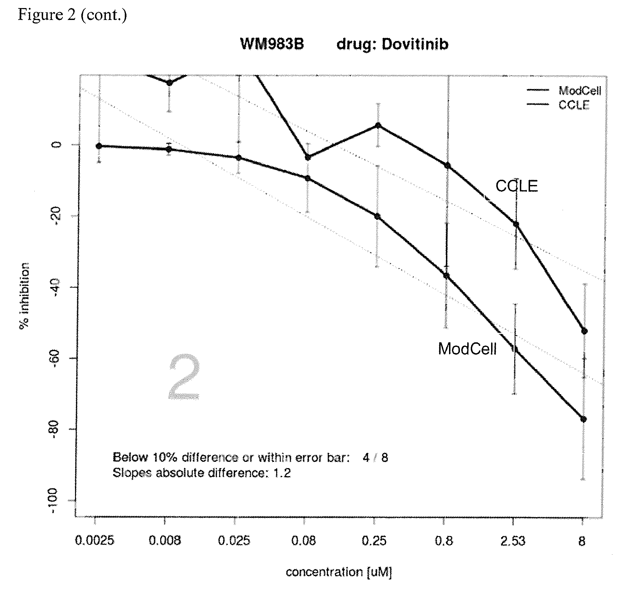

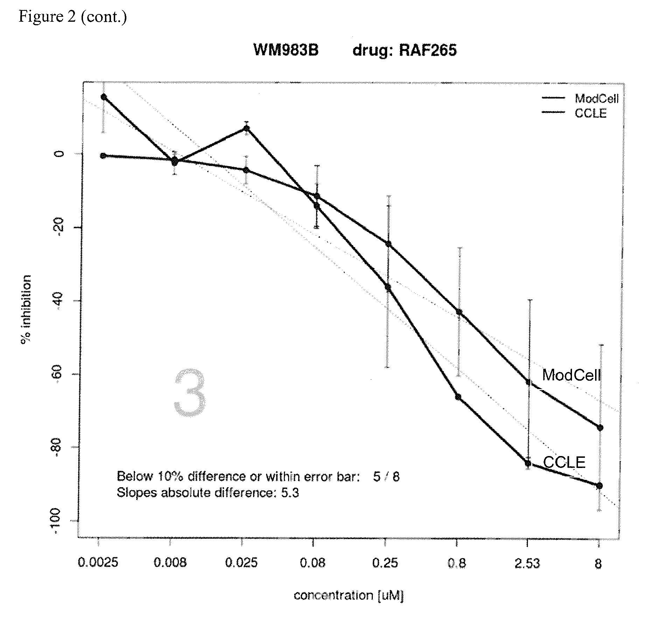

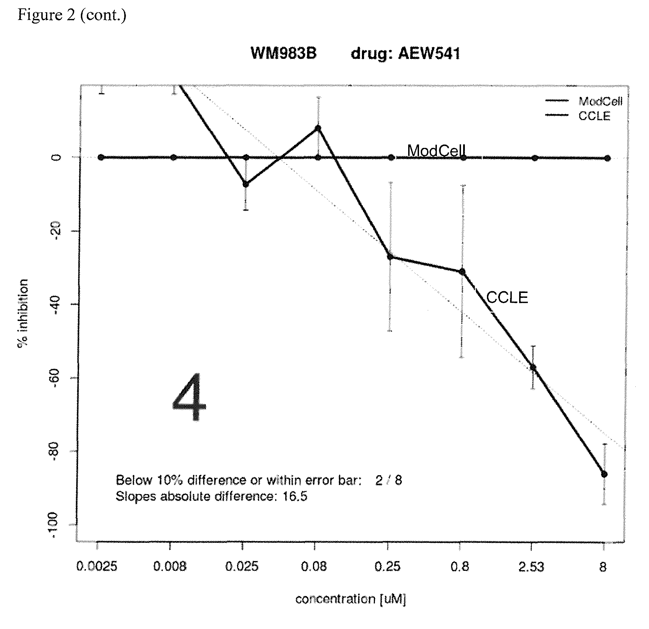

Examples of comparisons of measured and predicted growth inhibition curves of all accuracy categories are shown in Fig. 2. In total, predicted growth inhibition curves show very good accuracy in 103 cases (47.7%), good accuracy in 26 (12.0%) and 36 cases (16.7%), respectively, and poor accuracy in 51 cases (23.6%). Best accuracy was achieved for melanoma cell line HS695T with 75.0% very good and 25.0% good cases and with no predictions of category 4. The worst accuracy was found for the liver cancer cell line NCIH647 with only 41.7% very good and good cases, respectively.

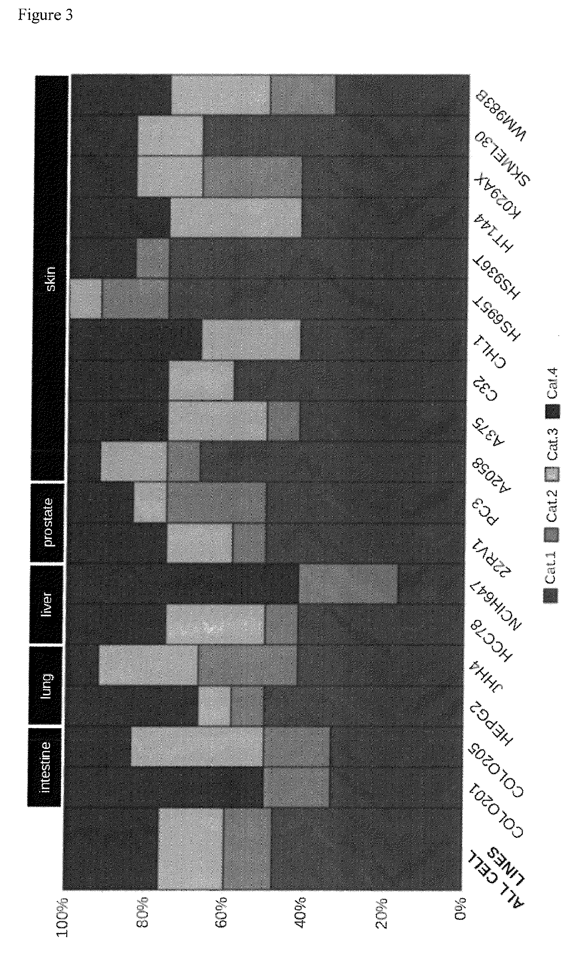

The results indicate that the modeling approach applied here is, in principle, independent of the tumor entity (Fig. 3). The reason is that the overall model represents the 'normal' biology of a somatic cell, and is then individualized to represent a specific tumor of a specific patient to allow the prediction of an optimized drug treatment based on the individual functional changes in the network.

The precise quantitative predictions of pharmacological profiles are often not necessary for clinical application. However, a decision for the selection of a single drug or drug combination a patient will respond/non respond to is essential. For this reason, the predicted growth inhibition curves were used to calculate IC50 values for every drug-cell line combination. Cell lines were then classified as sensitive (IC50<2µM) or resistant (IC50>2µM) for every drug based on predicted IC50 values and measured IC50.

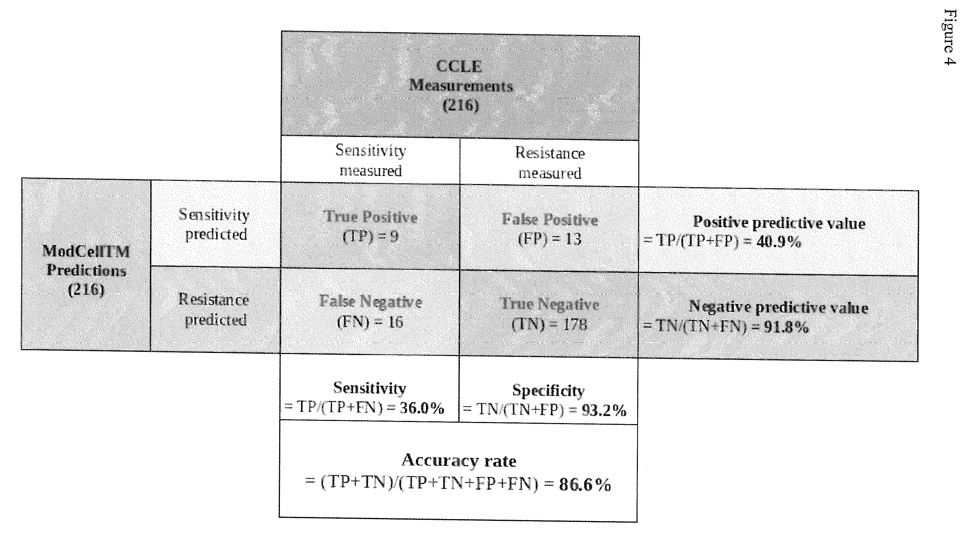

In total, according to the 216 CCLE measurements, 191 cell lines were classified as resistant to a particular drug and only 25 cell lines were classified as sensitive. 9 of these cell lines were also predicted as sensitive (true positives, TP) by the kinetic model using the IC50 classification, whereas 16 of them were wrongly predicted as resistant (false negatives, FN) leading to a sensitivity (true positive rate) of 36%. In contrast, 178 cell lines were correctly predicted as resistant (true negatives, TN) and only 13 were falsely predicted as sensitive (false positives, FP) resulting in a specificity (true negative rate) of over 93%. Overall, using the IC50 value as sensitivity criterion the kinetic model correctly predicted sensitivity and resistance, respectively, in 187 of 216 cases, leading to an even improved accuracy rate of over 86% (Fig. 4) as compared to the complex 8 point response curve classification shown above.

### Example 3

It was further investigated how the model compares to predictions of treatment based on current single marker predictions (e.g. BRAF V600E). Vemurafenib (an analogue of the pre-clinically tested PLX4720) is approved as monotherapy for the treatment of BRAF V600E mutation positive metastatic melanomas. However, resistance to Vemurafenib frequently occurs due to receptor tyrosine kinase-mediated activation of alternative survival pathways, activated RAS-mediated reactivation of the MAPK pathway and increased signaling through RAF1. Although 7 of the 10 melanoma cell lines analyzed harbour a BRAF V660E mutation (A2058, A375, C32, HS695T, HT144, K029AX, WM983B), only 3 of them show sensitivity to Vemurafenib treatment as revealed by CCLE measurements. The kinetic model in contrast identified 4 of the 7 BRAF V600E-mutated melanoma cell lines correctly as resistant to Vemurafenib. This confirms that the use of a single biomarker is not sufficient to reliably predict treatment outcome but that complex functional network information from a model must be taken into account to really improve on treatment outcome.

### Example 4

By deep sequencing it is now possible to characterize a tumor in more detail and coupled with our modeling approach every tumor can now be handled as a unique disease that might be treated with a drug for the treatment from other disease indications.

We therefore simulated the effects of 73 additional molecularly targeted drugs (anti-cancer as well as non anti-cancer, see Table 1) in 10 melanoma cell lines. Simulation of drug effects and calculation of growth inhibition curves were performed as described above and the same IC50 threshold was used for classification of drug sensitivity.

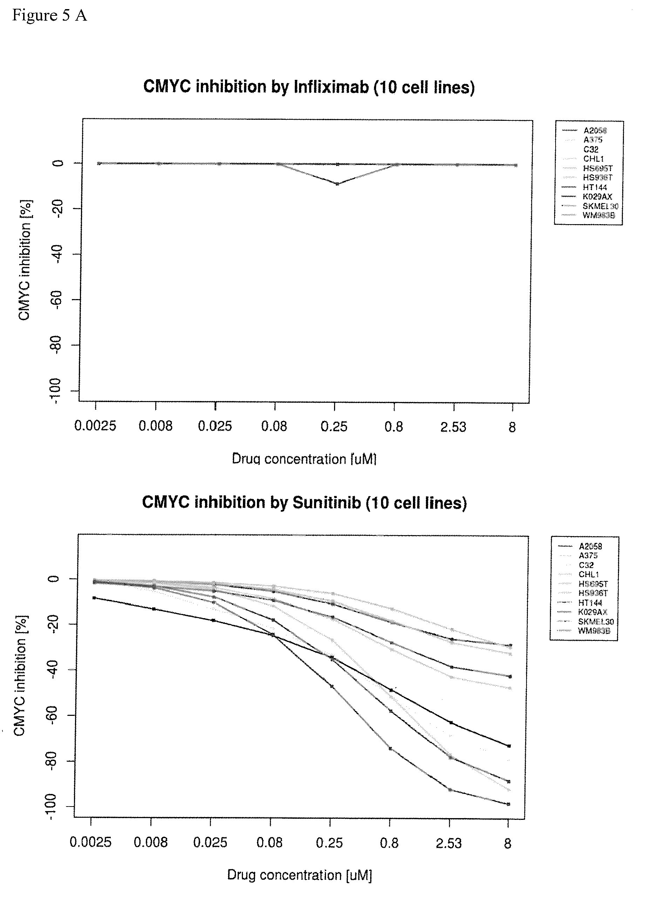

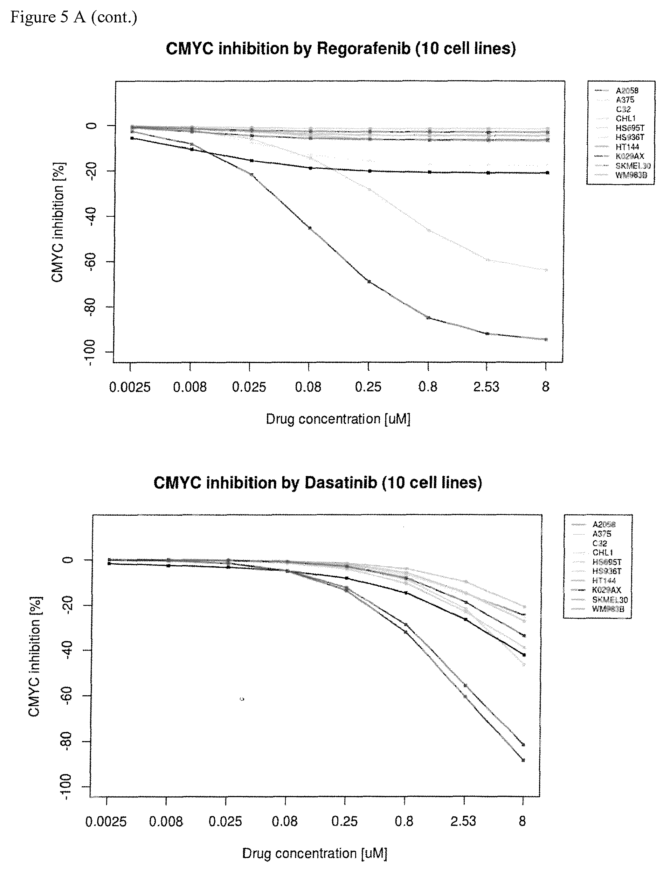

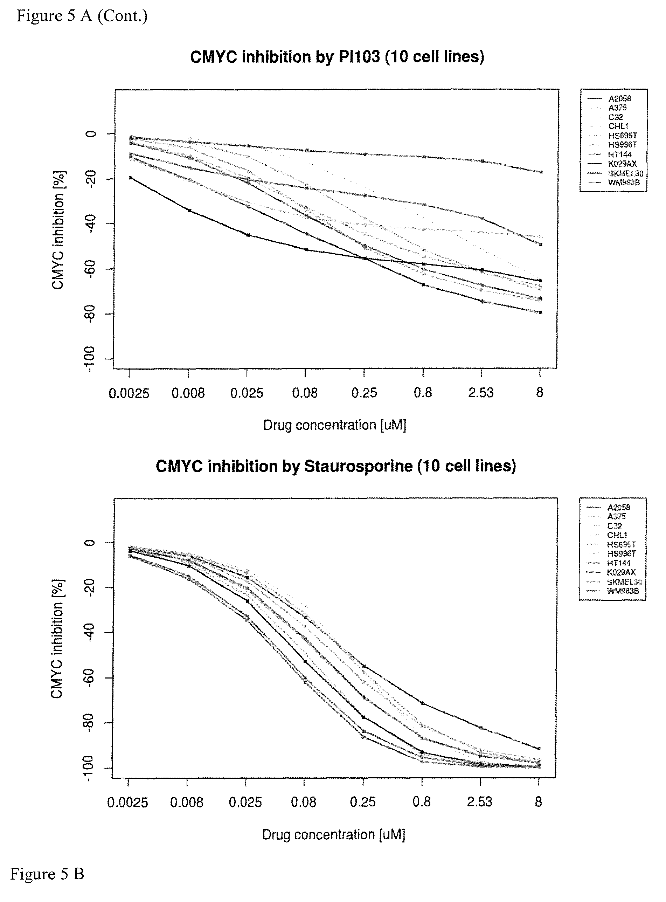

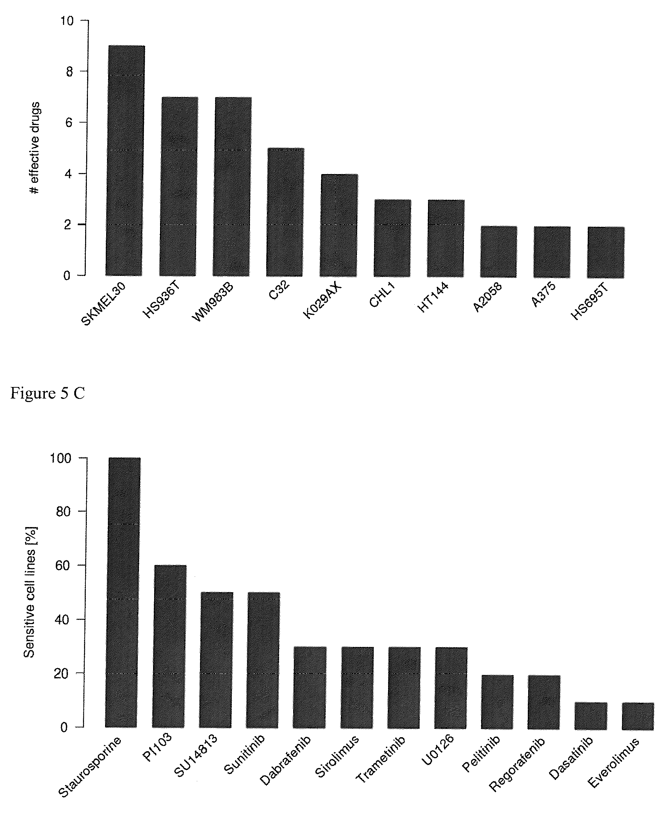

As shown in Fig. 5A,B the different drugs have very different impact on growth to the 10 melanoma cell lines. In total, we identified 12 different drugs that show significant inhibition of growth in at least one of the melanoma cell line. However, percentage of cell lines affected by the identified drugs varying from 10% (Dasatinib and Everolimus) to 100% (Staurosporine) a nonselective inhibitor of a diverse kinases and frequently used as positive control in cell culture experiments (Fig. 5C). These results demonstrate the effectiveness of drugs approved for non-skin cancer for melanoma. In particular, Sunitinib, a multi-receptor-tyrosine kinase inhibitor approved for renal cell carcinoma and Imatinib-resistant gastrointestinal tumor, significantly inhibits growth in melanoma cell lines C32 and HS936T, the cell lines A2058, A375, CHL1, HS695T, HT144 and K029AX show sensitivity to PI103, a dual PI3K-mTOR inhibitor. Also Regorafenib, a multi-kinase inhibitor approved for treatment of metastatic colorectal cancer, is effective in melanoma cell lines HS936T and SKMEL30. The melanoma cell line SKMEL also shows high sensitivity to Dasatinib, a multi BCR/Ab1 and SRC family tyrosine kinase inhibitor approved for first line use in patients with leukemia.

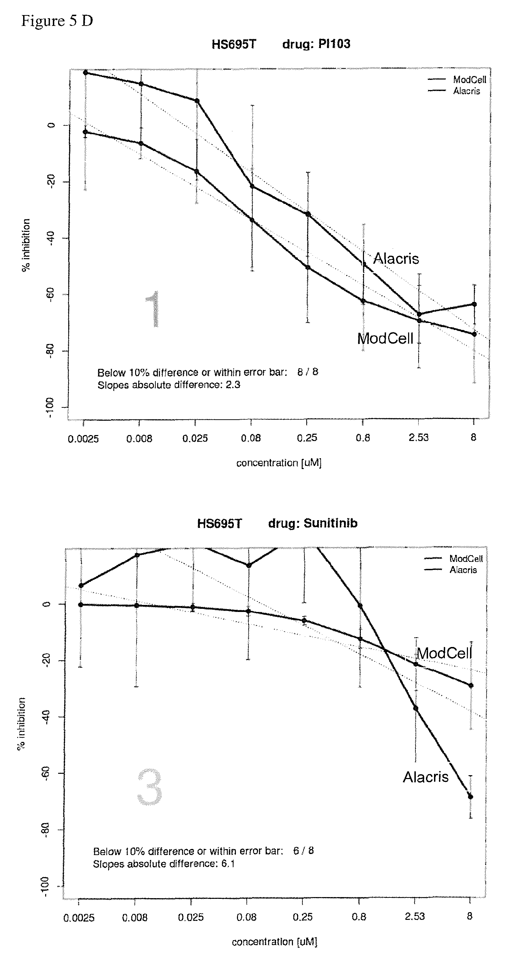

To validate our predictions we performed pharmacological characterization via cell culture experiments for a drug we found effective in cell line HS695T (PI103) and a drug that we identified as ineffective (Sunitinib). As shown in Fig. 5D sensitivity of cell line HS695T to PI103 was predicted with very good accuracy (accuracy category 1) and resistance of cell line HS695T to Sunitinib with good accuracy (accuracy category 3). Independently, the good prediction accuracy is confirmed by comparison of IC50 values. According to IC50 values determined by cell culture experiments (PI103: 0.83µM; Sunitinib: 4.7µM) and to predicted IC50 values (PI103: 0.24µM; Sunitinib 8µM) the cell line HS695T is sensitive to PI103, but resistant to Sunitinib. Using the herein described kinetic model, we are able to identify at least one molecular targeted drug for each of the cell lines that effectively inhibits cell growth according predicted IC50 values (PI103 in A2058, Everolimus, Sirolimus, SU14183 and Sunitinib in C32, PI103 in HS695T and PI103, SU14183 and Sunitinib in K029AX; Supplement Fig. 3; Supplement Tab. 4). Hence, the repositioning of cancer drugs approved for a particular tumor type provides a new rationale for effective cancer treatment in other cancer types that have relevant mutational profiles.

### Example 5

As shown above, from the 85 drugs we have tested *in silico* only Staurosporine and the compound PI103 have been identified as an effective monothereapy in melanoma cell line HS695T. In this example, we investigated whether it is possible to identify a combination of 2 drugs that effectively inhibits growth of cell line HS695T.

This was done by simulating all drug combinations using a concentration of 8µM for each drug (to reduce the number of combinations, we took only the drugs into account which showed at least a small effect on growth according to predicted maximum inhibition value). In total, 19 different drugs were taken into account leading to 171 different drug combinations. To evaluate the effects of drug combinations compared to corresponding single drugs we calculated the ratios of the predicted maximum inhibition values of drug combinations vs. the sum of the predicted maximum inhibition values of corresponding single drugs.

Interestingly, we identified drug combinations that show additive effects (ratio>0.95), drug combinations that show no or only little additive effects and even drug combinations that show negative effects, meaningful that a drug combination that show a higher maximum inhibition value (i.e. weaker inhibition) than at least one of the corresponding single drugs.

Combination of Dabrafenib and Midostaurin for example lead to a predicted maximum inhibition value of -62.73 showing additive effects of the single drugs (with predicted maximum inhibition values of -22.07 and -36.54, respectively). Combination of Pelitinib and Sunitinib on the other hand shows a maximum inhibition value of -29.58 which lies only marginally under the maximum inhibition value of -29.51 that is reached by treatment with Sunitinib alone. As Dovitinib, shows a predicted maximum inhibition value of -20.16, whereas in combination with Tozasertib the maximum inhibition is -10.32, combination of these 2 drugs is not effective.

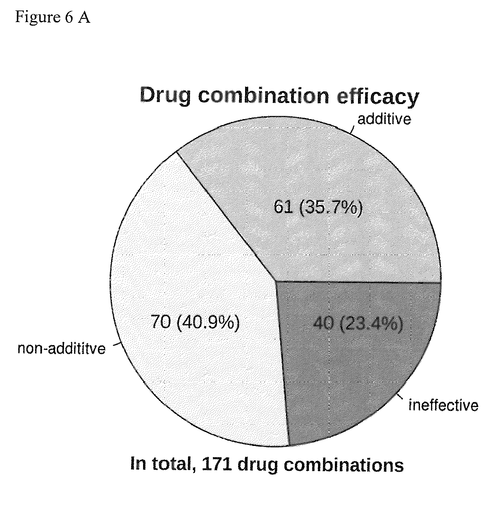

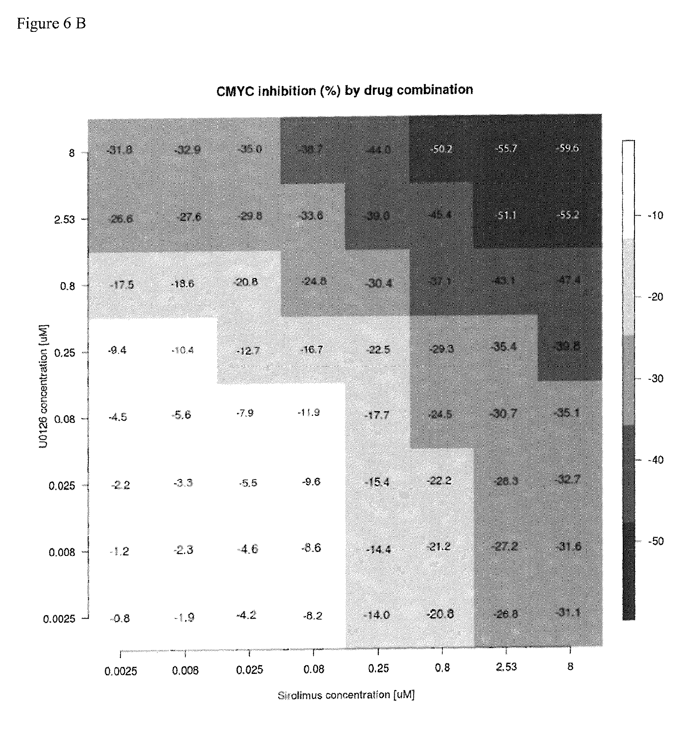

In total, we identified additive effects for 61 drug combinations (35.7%), 70 non-additive drug combinations (40.9%) and 40 drug combinations (23.4%) were classified as ineffective (Fig. 6A). The combination of Sirolimus and U0126 also showed additive effects (Fig. 6B) and was found as one of the most effective drug combinations with a predicted maximum inhibition value of -59.63.

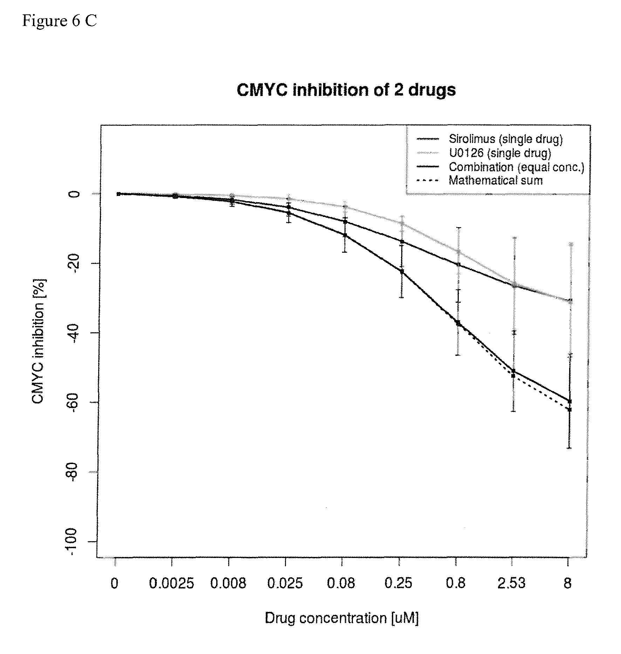

We therefore further simulated the effects of combinations of Sirolimus and U0126 in a dose dependent manner. As shown in Fig. 6C high inhibition of growth (over 50%) can be achieved by various combinations of concentrations of the single drugs (e.g., 0.8µM Sirolimus+8µM U0126 or 2.53µM Sirolimus+2.53µM U0126) showing how modeling by the herein described model can not only identify effective drug combinations but also help to adjust drug concentrations to individual needs.

To validate the predictions of combinatorial effects of Sirolimus and U0126, we performed cell culture experiments to determine growth inhibition curves using 3 different concentration ranges of the 2 drugs: i) concentration of Sirolimus fixed to 2.5 nM and concentration of U0126 ranging from 8 µM to 2.5 nM by 3.16-fold dilutions, ii) concentration of U0126 fixed to 2.5 nM and concentration of U0126 ranging from 8 µM to 2.5 nM by 3.16-fold dilutions and iii) equal concentrations of Sirolimus and U0126 ranging from 8 µM to 2.5 nM by 3.16-fold dilutions. Predicted growth inhibition curves were compared to measured growth inhibition curves as previously described.

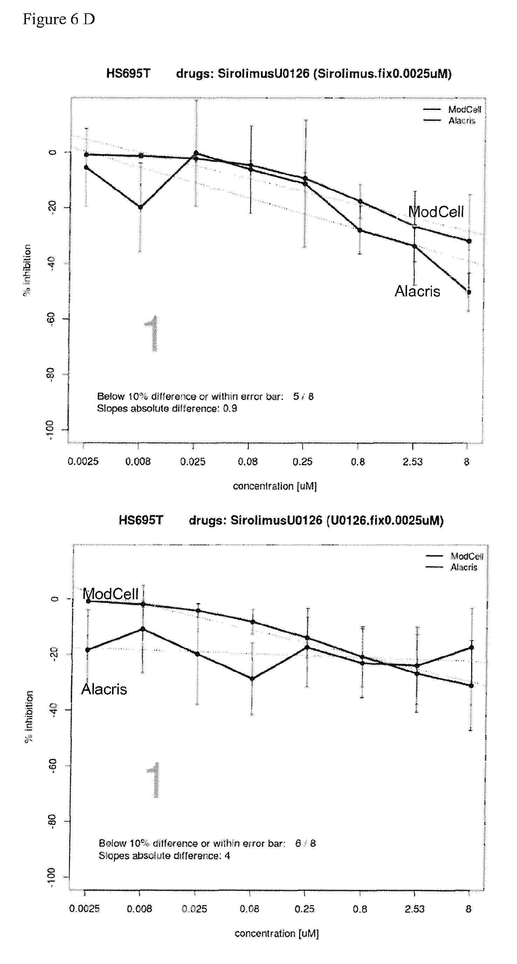

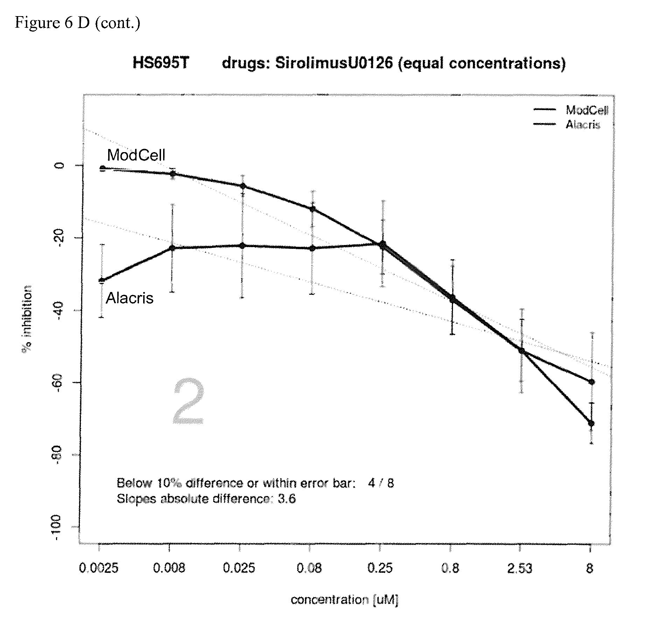

As shown in Fig. 6D combinatorial effects of Sirolimus and U0126 were predicted with very good and good accuracy, respectively and show a 4-fold additive effect toward inhibiting cell growth more efficiently. This application can therefore provide a highly efficient platform for prioritizing cell and clinical studies. This is critical as combinatory studies are, due the large number of possible combinations plus variation in drug concentrations highly demanding both financially as well as experimentally.

### FIGURE CAPTIONS

**Fig. 1****: Cancer cell line specific models.** A) Within the selected 18 cancer cell lines identified mutations that have been introduced into the cancer cell line specific models as gain-of-function mutations. B) Predictions of changes in selected components of the model for selected cancer cell lines modeled according to their mutation patterns and expression profiles. The diagram shows log2-ratios of the cell line states vs. the corresponding control states (Material & Methods) for different ligand-receptor-complexes of various cancer relevant pathways of the model indicating the activation status of these pathways.
**Fig. 2****: Comparison of growth inhibition curves**. The diagrams show growth inhibition curves predicted by ModCell (red) and corresponding curves determined by CCLE (blue).
For quality assessment, the number of matched data points as well as slopes of regression lines (dotted) of growth inhibition curves are shown. For each of the 4 prediction accuracy categories one example is shown. Predicted responses of cell line WM983B to Selumetinib show very good agreement to responses described by CCLE (8/8 overlapping data points and a slope difference of 3.0; accuracy category 1). Good prediction accuracy is observed for the responses of cell line WM983B to Dovitinib and RAF265 (4/8 and 5/8 overlapping data points and a slope difference of 1.2 and 5.3, respectively; accuracy category 2 and 3, respectively). Prediction of growth inhibition by AEW541 in cell line WM983B in contrast show only poor accuracy (2/8 overlapping data points and slope difference of 16.5; accuracy category 4). Error bars display the standard deviation.
**Fig. 3****: Overview of prediction accuracy**. The diagram shows the percentages for each prediction accuracy category averaged over all selected cancer cell lines and for single cancer cell lines, respectively. Accuracy categories (Cat.1: very good accuracy, dark green; Cat.2/3: good accuracy, green and light green, respectively; Cat.4: poor accuracy, red) are defined as described in Material & Methods and were determined by comparison of growth inhibition curves.
**Fig. 4****: Accuracy rate of predictions using IC50 values.** The diagram shows the number of correctly and wrongly predicted sensitive and resistant cell lines, respectively, by comparing predicted IC50 values and IC50 values determined by CCLE. False predictions, wrongly predicted sensitivity (false positives, FP) as well as wrongly predicted resistance (false negatives, FN) in red; right predictions, correctly predicted sensitivity (true positives, TP) as well as correctly predicted resistance (true negatives, TN) in green.
**Fig. 5****: Prediction of drug effects in selected melanoma cell lines.** A) Growth inhibition curves predicted by ModCell for exemplary drugs in all 10 melanoma cell lines. Error bars are omitted for clarity. B) The number of identified effective drugs in selected melanoma cell lines according to predicted IC50 values. C) The percentage of melanoma cell lines sensitive to a corresponding drug according to predicted IC50 values. Drugs that were not found to be effective in any cell line (n=61) are not shown. D) Validation of predicted drug response of cell line HS695T to PI103 and to Sunitinib. Accuracy of predictions are classified by comparison of growth inhibition curves predicted by ModCell (red) and of growth inhibition curves measured by cell culture experiments (blue) as described in Material & Methods. Error bars display the standard deviation.
**Fig. 6****: Prediction of effects of drug combinations.** A) Distribution of drug combination efficacy, separated in 3 categories: additive, non-additive, and inefficient. Additive are drug combinations that show an inhibitory effect similar to the sum of corresponding single drugs (combination-to-sum-ratio > 0.95). Ineffective are drug combinations that show an inhibitory effect that is smaller than one of the single drugs. Non-additive are drug combinations that show a better efficacy than ineffective drug combinations, but are not additive. B) Predicted growth inhibition by Sirolimus (blue), U0126 (green) and combination of Sirolimus and U0126 (black). Theoretical growth inhibition curve as sum of growth inhibition curves of single drug treatments in dashed black. Error bars display the standard deviation. C) Predicted inhibition of growth of cell line HS695T by combinatorial treatment with U0126 and Sirolimus. D) Validation of predicted growth inhibition curves. Accuracy of predictions are classified by comparison of growth inhibition curves predicted by ModCell (red) and of growth inhibition curves measured by CCLE (blue) as described in Material & Methods. Error bars display the standard deviation.

## Claims

1. Method for identifying a therapeutic drug combination against a cancer, wherein the cancer comprises at least two alterations in at least two different, but crosstalking signaling pathways, the method comprising the steps of:
a. providing a kinetic model of a biological network for said cancer comprising the at least two different, but crosstalking signaling pathways, wherein the kinetic model is generated by choosing a network topology, wherein the nodes of said topology represent biological entities selected from the group comprising genes, transcripts, peptides, proteins, protein modification states, small molecules, complexes, metabolites and modifications thereof, and the edges of said topology represent interactions between said entities, assigning kinetic laws and kinetic constants to the interactions and assigning concentrations to the biological entities, such that the kinetic model reflects the genome, epi-genome, proteome and/or transcriptome of said cancer,
b. selecting test combinations from a plurality of known drugs, each test combination comprising at least two drugs,
c. simulating the effect of each test combination on the biological network, thereby
d. identifying from said test combinations a drug combination that acts against said cancer.

2. Method for predicting the response of a cancer to a therapeutic drug combination, wherein the cancer comprises at least two alterations in at least two different, but crosstalking signaling pathways, the method comprising the steps of:
a. providing a kinetic model of a biological network for said cancer comprising the at least two different, but crosstalking signaling pathways, wherein the kinetic model is generated by choosing a network topology, wherein the nodes of said topology represent biological entities selected from the group comprising genes, peptides, nucleic acids, proteins, small molecules, complexes, metabolites and modifications thereof, and the edges of said topology represent interactions between said entities, assigning kinetic laws and kinetic constants to the interactions and assigning concentrations to the biological entities, such that the kinetic model reflects the genome, epigenome proteome and/or transcriptome of said cancer,
b. providing a drug combination comprising at least two drugs, preferably one for each of the at least two different signaling pathways,
c. simulating the effect of the drug combination on the biological network, thereby
d. determining whether the cancer is responsive to the drug combination.

3. Method of claim 1 or 2, wherein the at least two alterations in the at least two signaling pathways are selected from the group comprising mutations, overexpression, fusions, epigenetic changes and insertions.

4. Method of claims 1 to 3, wherein crosstalk between the signaling pathways occurs via a protein shared by the signaling pathways, transmembrane crosstalk, crosstalk in transcriptional activation, or crosstalk on a transcriptional level.

5. Method of claims 1 to 4, wherein the kinetic model reflects crosstalk between the signaling pathways by biological entities shared by the signaling pathways.

6. Method of claims 1 to 5, wherein the at least two alterations are determined by analyzing at least parts of the genome, epigenome, transcriptome and/or proteome of said cancer.

7. Method of claim 6, wherein the genome, epigenome and/or transcriptome is analyzed by sequencing, preferably next-generation sequencing.

8. Method of claims 1 to 7, wherein the alterations are gain of function, loss of function or gene-overexpression like.

9. The method of claims 1 to 8, wherein the effect of each candidate is evaluated by entities reflecting cell survival, or cell proliferation.

10. The method of claims 1 to 9, further simulating the effect of a single drug of one of the candidates or the drug combination identified for said cancer on the biological network and comparing the effectiveness of one of the candidates or the drug combination identified for said cancer to the sum of the effectiveness of the single drugs corresponding to said combination.

11. The method of claims 1 to 10, wherein the at least two drugs have a known pharmacologic profile.

12. The method of claims 1 to 11, wherein the at least two drugs have a known IC50 value.

13. The method of claims 1 to 12, wherein the at least two drugs are targeted mechanistic drugs, preferably selected from the group of tyrosinase kinase inhibitors and monoclonal antibodies.

14. The method of claims 1 to 13, wherein the biological network represents a human or a part thereof, a tissue, a cell line, one or more cells or a mixture thereof.

15. The method of claims 1 to 14, wherein the identified drug combination is further tested in a cancer-specific cell line, a xenograft model and/or in clinical trials.
